(19) Europäisches Patentamt
European Patent Office
Office européen des brevets

(11) **EP 4 681 645 A1**

(12) **EUROPEAN PATENT APPLICATION**
published in accordance with Art. 153(4) EPC

(43) Date of publication:
**21.01.2026 Bulletin 2026/04**

(21) Application number: **23927577.9**

(22) Date of filing: **06.11.2023**

(51) International Patent Classification (IPC):
***A61B 6/00*** *(2024.01)*

(52) Cooperative Patent Classification (CPC):
**A61B 6/505; A61B 6/00; A61B 6/032; A61B 6/482**

(86) International application number:
**PCT/JP2023/039909**

(87) International publication number:
**WO 2024/189963 (19.09.2024 Gazette 2024/38)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **13.03.2023 JP 2023038539**

(71) Applicant: **FUJIFILM Corporation
Tokyo 106-8620 (JP)**

(72) Inventor: **KAWAMURA, Takahiro
Ashigarakami-gun, Kanagawa 258-8538 (JP)**

(74) Representative: **Dehns Germany Partnerschaft
mbB
Theresienstraße 6-8
80333 München (DE)**

(54) **IMAGE PROCESSING DEVICE, METHOD, AND PROGRAM**

(57) A processor is configured to acquire two radiation images acquired by imaging a subject including a bone part and a soft part with radiation having different energy distributions, derive a bone part image including a microstructure inside the bone part based on the two radiation images, and derive a likelihood of osteoporosis based on the bone part image or a partial image including a bone of interest in the bone part image.

FIG. 3

IMAGE PROCESSING APPARATUS — 10
IMAGE ACQUISITION UNIT — 21
SCATTERED RAY REMOVAL UNIT — 22
BONE PART IMAGE DERIVATION UNIT — 23
SEMENTATION UNIT — 24
LIKELIHOOD DERIVATION UNIT — 25
TRAINED MODEL — 25A
DISPLAY CONTROL UNIT — 26

EP 4 681 645 A1

**Description**

**BACKGROUND OF THE INVENTION**

1. Field of the Invention

The present invention relates to an image processing apparatus, an image processing method, and an image processing program.

2. Description of the Related Art

Fractures of a femur and a vertebra that occur as a complication of osteoporosis are likely to cause a patient to be bedridden. It is known that a five-year survival rate for bedridden patients is lower than a five-year survival rate for cancer. Since the number of patients with osteoporosis is increasing worldwide, it is expected that medical expenses and nursing care expenses can be reduced if osteoporosis can be detected and treated early. Therefore, various methods for evaluating osteoporosis have been proposed.

For example, in CN113129286A, a method of detecting osteoporosis by acquiring a bone density image having a uniform size from a bone density image acquired by a dual X-ray absorptiometry (DXA) method or a quantitative computed tomography (CT) method using a CT image and performing feature identification on the bone density image having a uniform size has been proposed.

**SUMMARY OF THE INVENTION**

On the other hand, the bone part includes a trabecular structure of a cancellous bone inside the bone part. The trabeculae have a microstructure, and the trabeculae are densely present in a healthy person, but the density of the trabeculae is low in a patient with osteoporosis. Therefore, accurately understanding the state of sparse-and-dense pattern of the microstructure of the trabeculae leads to highly accurate detection of osteoporosis. However, the bone density image acquired by the above-described DXA method or quantitative CT method has high spatial resolution. For example, the spatial resolution of the bone density image obtained by the DXA method is about 0.6 to 2.0 mm/pixel, and the spatial resolution of the bone density image obtained by the quantitative CT method is about 0.5 to 1.0 mm/pixel. In the bone density image acquired by the DXA method or the quantitative CT method having such spatial resolution, the microstructure of the trabecula is not sufficiently depicted in the image, and thus it is not possible to accurately determine osteoporosis.

The present disclosure has been made in view of the above circumstances, and an object of the present disclosure is to enable simple and accurate discrimination of osteoporosis.

An image processing apparatus according to the present disclosure comprises at least one processor, in which the processor is configured to:

acquire two radiation images acquired by imaging a subject including a bone part and a soft part with radiation having different energy distributions;
derive a bone part image including a microstructure inside the bone part based on the two radiation images; and
derive a likelihood of osteoporosis based on the bone part image or a partial image including a bone of interest in the bone part image.

The "partial image including a bone of interest" may be an image of only the bone of interest or may be an image including the bone of interest and a region around the bone of interest.

In the image processing apparatus according to the present disclosure, the bone part image may include a trabecular structure of cancellous bone included in the bone part.

In addition, in the image processing apparatus according to the present disclosure, the trabecular structure of the cancellous bone may be depicted in the bone part image.

The term "depicted" means that the trabecular structure of the cancellous bone can be visually recognized in the bone part image. In order to depict the trabecular structure of the cancellous bone, it is necessary that the spatial resolution per pixel of the bone part image is equal to or lower than the trabecular structure of the cancellous bone, and for example, the spatial resolution of the bone part image is equal to or lower than 0.2 mm/pixel, preferably equal to or lower than 0.15 mm/pixel, and more preferably equal to or lower than 0.1 mm/pixel.

In addition, in the image processing apparatus according to the present disclosure, the processor may be configured to extract the partial image from at least one of the two radiation images or the bone part image.

In addition, in the image processing apparatus according to the present disclosure, the processor may be configured to extract at least one of the bones of interest from at least one of the two radiation images or the bone part image, and extract the partial image based on a centroid of the bones of interest.

In addition, in the image processing apparatus according to the present disclosure, the processor may be configured to function as a trained model in which machine learning is performed to output a likelihood of osteoporosis in response to an input of the bone part image or the partial image.

In addition, in the image processing apparatus according to the present disclosure, the processor may be configured to function as a trained model that has undergone machine to output the likelihood of osteoporosis in response to an input of imaging conditions of the two radiation images in addition to the bone part image or the partial image.

In addition, in the image processing apparatus according to the present disclosure, the processor may be configured to derive a likelihood of osteoporosis based on a statistical value of pixel values of the bones of interest included in the bone part image or the partial image.

In addition, in the image processing apparatus according to the present disclosure, the statistical value may be at least one of an average value, a standard deviation, a maximum value, or a minimum value of pixel values in each of the bone of interest and a peripheral region of the bone of interest.

In addition, in the image processing apparatus according to the present disclosure, the processor may be configured to derive the likelihood by performing a weighting operation on a plurality of statistical values among an average value, a standard deviation, a maximum value, and a minimum value of pixel values in each of the bone of interest and the peripheral region of the bone of interest.

In addition, in the image processing apparatus according to the present disclosure, the weight of a weighting operation may be derived by machine learning.

In addition, in the image processing apparatus according to the present disclosure, the processor may be configured to notify the likelihood.

In addition, in the image processing apparatus according to the present disclosure, the processor may be configured to issue a warning in a case where the likelihood is equal to or greater than a predetermined threshold value.

In addition, in the image processing apparatus according to the present disclosure, the processor may be configured to determine a priority of a treatment for osteoporosis based on the likelihood, and issue a notification of the priority.

In addition, in the image processing apparatus according to the present disclosure, the processor may be configured to remove scattered ray components included in the two radiation images to derive the bone part image.

In addition, in the image processing apparatus according to the present disclosure, the processor is configured to:

derive, in a soft region including only the soft part of the two radiation images, a characteristic of the soft part related to an attenuation of the radiation based on the two radiation images;

derive, in a bone region including the bone part of the two radiation images, a characteristic of the soft part based on the characteristic of the soft part derived in the soft region around the bone region; and

derive, as the bone part image, an image in which the bone part is emphasized based on the characteristic of the soft part in at least a region of the subject in the two radiation images.

In the image processing apparatus according to the present disclosure, the bone of interest may be a femur or a vertebra.

According to the present disclosure, there is provided an image processing method comprising:

via a computer,

acquiring two radiation images acquired by imaging a subject including a bone part and a soft part with radiation having different energy distributions;

deriving a bone part image including a microstructure inside the bone part based on the two radiation images; and

deriving a likelihood of osteoporosis based on the bone part image or a partial image including a bone of interest in the bone part image.

According to the present disclosure, there is provided an image processing program causing a computer to execute a process comprising:

acquiring two radiation images acquired by imaging a subject including a bone part and a soft part with radiation having different energy distributions;

deriving a bone part image including a microstructure inside the bone part based on the two radiation images; and

deriving a likelihood of osteoporosis based on the bone part image or a partial image including a bone of interest in the bone part image.

According to the present disclosure, osteoporosis can be simply and accurately discriminated.

## BRIEF DESCRIPTION OF THE DRAWINGS

Fig. 1 is a schematic block diagram showing a configuration of a radiography system to which an image processing apparatus according to a first embodiment of the present disclosure is applied.
Fig. 2 is a diagram showing a schematic configuration of the image processing apparatus according to the first embodiment.
Fig. 3 is a diagram showing a functional configuration of the image processing apparatus according to the first embodiment.
Fig. 4 is a diagram showing a bone part image.
Fig. 5 is a diagram showing a result of segmentation.
Fig. 6 is a diagram showing a schematic configuration of a neural network used in the present embodiment.
Fig. 7 is a diagram showing teacher data used for constructing a trained model in the first embodiment.
Fig. 8 is a diagram showing an example of a trained model used in the first embodiment.
Fig. 9 is a diagram showing an example of a display screen.
Fig. 10 is a flowchart of learning processing performed in the first embodiment.
Fig. 11 is a graph showing a profile of a radiation image acquired in the present embodiment.
Fig. 12 is a graph showing profiles of bone part images derived from two radiation images acquired in the present embodiment.
Fig. 13 is a graph showing a profile of a bone part image corresponding to a bone density image acquired by a quantitative CT method.
Fig. 14 is a graph showing a profile of a bone part image corresponding to a bone density image acquired by the DXA method.
Fig. 15 is a diagram for describing a section indicated by a horizontal axis of the graphs shown in Figs. 11 to 14.
Fig. 16 is a diagram showing a functional configuration of an image processing apparatus according to a second embodiment.
Fig. 17 is a diagram showing teacher data used for constructing a trained model in the second embodiment.
Fig. 18 is a diagram showing an example of a trained model used in the second embodiment.
Fig. 19 is a diagram showing a functional configuration of an image processing apparatus according to a third embodiment.
Fig. 20 is a diagram showing an example of a display screen including a warning.
Fig. 21 is a diagram showing an example of a display screen including a priority of a treatment.
Fig. 22 is a diagram showing a functional configuration of a bone part image derivation unit of the image processing apparatus according to a fourth embodiment.
Fig. 23 is a diagram schematically showing processing performed by a bone part image derivation unit of the image processing apparatus according to the fourth embodiment.
Fig. 24 is a flowchart showing a process performed in the fourth embodiment.

## DESCRIPTION OF THE PREFERRED EMBODIMENTS

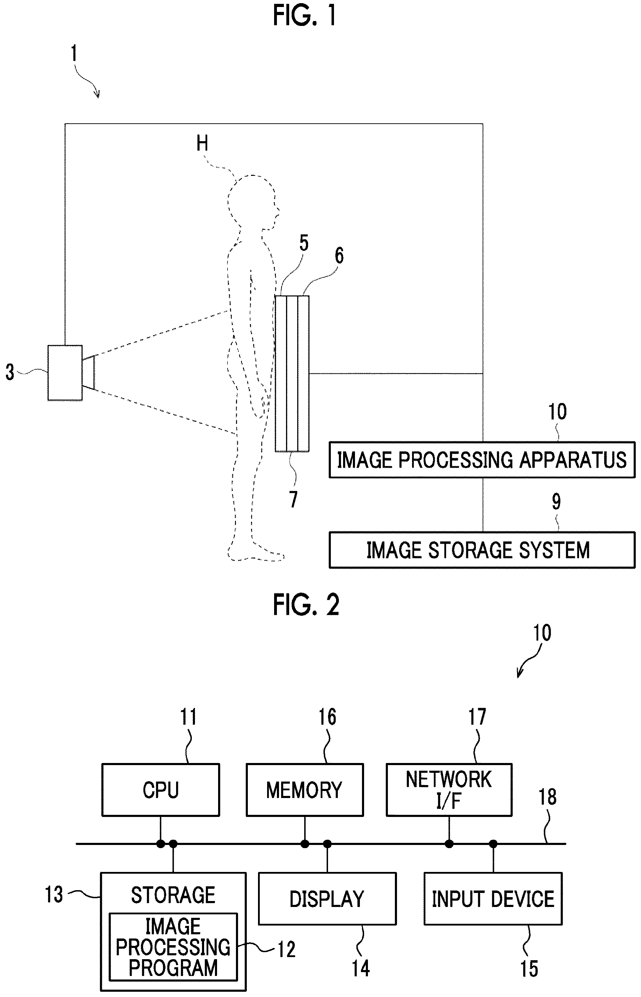

Hereinafter, embodiments of the present disclosure will be described with reference to the drawings. Fig. 1 is a schematic block diagram showing a configuration of a radiation image imaging system to which an image processing apparatus according to a first embodiment of the present disclosure is applied. As shown in Fig. 1, the radiography system according to the present embodiment comprises an imaging apparatus 1 and an image processing apparatus 10 according to the first embodiment.

The imaging apparatus 1 is an imaging apparatus for performing energy subtraction by a so-called one-shot method for converting radiation, such as X-rays, emitted from a radiation source 3 and transmitted through a subject H into energy and irradiating a first radiation detector 5 and a second radiation detector 6 with the converted radiation. During the imaging, as shown in Fig. 1, the first radiation detector 5, a radiation energy conversion filter 7 made of a copper plate or the like, and the second radiation detector 6 are disposed in order from a side closest to the radiation source 3, and the radiation source 3 is driven. It should be noted that the first and second radiation detectors 5 and 6 are closely attached to the radiation energy conversion filter 7.

As a result, in the first radiation detector 5, a first radiation image G1 of the subject H by low-energy radiation also including so-called soft X-rays is acquired. Further, in the second radiation detector 6, a second radiation image G2 of the subject H by high-energy radiation from which the soft ray is removed is acquired. Note that both the first and second radiation images G1 and G2 are two-dimensional images that are transmission images of the subject acquired by simple imaging in which the radiation is emitted to the subject H once. Thus, both the first and second radiation images G1 and G2 are simple radiation images. In addition, in the first and second radiation images G1 and G2, the higher the brightness (that is, the lower the density), the larger the pixel value.

The first and second radiation detectors 5 and 6 can perform recording and reading-out of the radiation image repeatedly. A so-called direct-type radiation detector that directly receives emission of the radiation and generates an electric charge may be used, or a so-called indirect-type radiation detector that converts the radiation into visible light and then converts the visible light into an electric charge signal may be used. In addition, as a method for reading out a radiation image signal, it is desirable to use a so-called thin film transistor (TFT) readout method in which the radiation image signal is read out by turning a TFT switch on and off, or a so-called optical readout method in which the radiation image signal is read out by emission of read out light, but other methods may also be used without being limited to these methods.

The image processing apparatus 10 is connected to the image storage system 9 via a network (not shown).

The image storage system 9 is a system that stores image data of the radiation image captured by the imaging apparatus 1. The image storage system 9 extracts an image corresponding to a request from the image processing apparatus 10 from the stored radiation image and transmits the extracted image to a request source device. Specific examples of the image storage system 9 include picture archiving and communication systems (PACS).

Next, the image processing apparatus according to the first embodiment will be described. First, a hardware configuration of the image processing apparatus according to the present embodiment will be described with reference to Fig. 2. As shown in Fig. 2, the image processing apparatus 10 is a computer, such as a workstation, a server computer, and a personal computer, and comprises a central processing unit (CPU) 11, a non-volatile storage 13, and a memory 16 as a transitory storage region. In addition, the image processing apparatus 10 comprises a display 14, such as a liquid crystal display, an input device 15, such as a keyboard and a mouse, and a network interface (I/F) 17 connected to a network (not shown). The CPU 11, the storage 13, the display 14, the input device 15, the memory 16, and the network I/F 17 are connected to a bus 18. Note that the CPU 11 is an example of a processor according to the present disclosure.

The storage 13 is implemented by a hard disk drive (HDD), a solid state drive (SSD), a flash memory, or the like. An image processing program 12 installed in the image processing apparatus 10 is stored in the storage 13 as a storage medium. The CPU 11 reads out the image processing program 12 from the storage 13, develops the image processing program 12 in the memory 16, and executes the developed image processing program 12.

The image processing program 12 is stored in a storage device of a server computer connected to the network or in a network storage in an externally accessible state and is downloaded and installed on a computer that constitutes the image processing apparatus 10 in response to a request. Alternatively, the image processing program 12 is distributed by being recorded on a recording medium such as a digital versatile disc (DVD) or a compact disc read only memory (CD-ROM) and is then installed onto the computer that constitutes the image processing apparatus 10 from the recording medium.

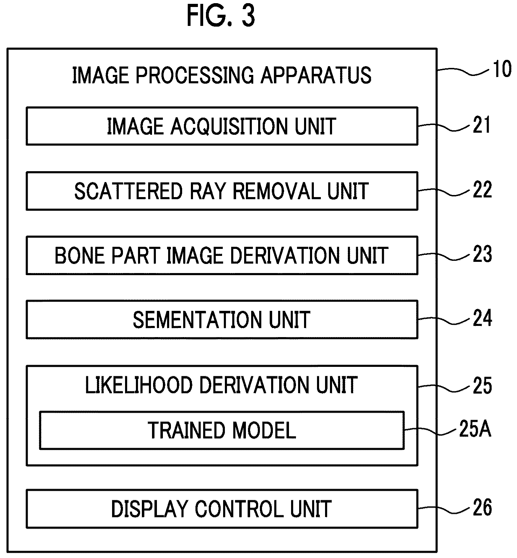

Next, a functional configuration of the image processing apparatus according to the first embodiment will be described. Fig. 3 is a diagram showing a functional configuration of the image processing apparatus according to the first embodiment. As shown in Fig. 3, the image processing apparatus 10 comprises an image acquisition unit 21, a scattered ray removal unit 22, a bone part image derivation unit 23, a segmentation unit 24, a likelihood derivation unit 25, and a display control unit 26. Then, the CPU 11 functions as the image acquisition unit 21, the scattered ray removal unit 22, the bone part image derivation unit 23, the segmentation unit 24, the likelihood derivation unit 25, and the display control unit 26 by executing the image processing program 12, and further functions as a trained model 25A described below.

The image acquisition unit 21 causes the imaging apparatus 1 to perform the imaging of the subject H to acquire, from the first and second radiation detectors 5 and 6, the first radiation image G1 and the second radiation image G2 which are frontal images of the vicinity of the crotch of the subject H, for example. In acquiring the first radiation image G1 and the second radiation image G2, imaging conditions are set, such as a radiation exposure dose, a tube voltage, a source image receptor distance (SID) which is a distance between the radiation source 3 and surfaces of the first and second radiation detectors 5 and 6, a source object distance (SOD) which is a distance between the radiation source 3 and a surface of the subject H, and the presence or absence of a scattered ray removal grid.

The SOD and the SID are used to calculate a body thickness distribution as described below. It is preferable to obtain the SOD by, for example, a time of flight (TOF) camera. It is preferable that the SID is acquired by, for example, a potentiometer, an ultrasound distance meter, or a laser distance meter.

The imaging condition may be set by an input from the input device 15 by an operator. The set imaging condition is stored in the storage 13.

In the present embodiment, the first and second radiation images G1 and G2 may be acquired by a program separate from the image processing program 12 and stored in the storage 13. In this case, the image acquisition unit 21 performs the acquisition by reading out the first and second radiation images G1 and G2 stored in the storage 13 from the storage 13 for processing.

Here, in the human bone, the cancellous bone having a dense structure inside a cortical bone covering an outside of the bone is present. The cancellous bone has a structure in which trabeculae having a thickness of about 150 $\mu$m are three-dimensionally connected (hereinafter, referred to as a trabecular structure). The first and second radiation images G1 and G2 acquired in the present embodiment include a trabecular structure of the cancellous bone included in the inside of the bone part. That is, the first and second radiation images G1 and G2 are high-definition images in which the trabecular structure of the cancellous bone is depicted such that the trabecular structure of the cancellous bone can be visually recognized. The spatial resolution of the first and second radiation images G1 and G2 is 0.2 mm/pixel or less, preferably 0.15 mm/pixel, and more preferably 0.1 mm/pixel or less so that the trabecular structure of the cancellous bone can be depicted.

Here, each of the first radiation image G1 and the second radiation image G2 includes a scattered ray component based on the radiation scattered in the subject H in addition to a primary ray component of the radiation transmitted through the subject H. Thus, the scattered ray removal unit 22 removes the scattered ray component from the first radiation image G1 and the second radiation image G2. For example, the scattered ray removal unit 22 may apply the method described in JP2015-043959A to remove the scattered ray component from the first radiation image G1 and the second radiation image G2. In a case where the method described in JP2015-043959A or the like is used, the derivation of the body thickness distribution of the subject H and the derivation of the scattered ray component for removing the scattered ray component are performed at the same time.

Hereinafter, the removal of the scattered ray component from the first radiation image G1 will be described, but the removal of the scattered ray component from the second radiation image G2 can also be performed in the same manner. First, the scattered ray removal unit 22 acquires a virtual model K of the subject H having an initial body thickness distribution T0(x,y). The virtual model K is data, which virtually represents the subject H, in which the body thickness according to the initial body thickness distribution TO(x,y) is associated with a coordinate position of each pixel of the first radiation image G1. The virtual model K of the subject H having the initial body thickness distribution TO(x,y) may be stored in advance in the storage 13. Further, a body thickness distribution T(x,y) of the subject H may be calculated based on the SID and the SOD included in the imaging condition. In this case, the body thickness distribution can be obtained by subtracting the SOD from the SID.

Next, the scattered ray removal unit 22 generates, based on the virtual model K, an image in which an estimated primary ray image obtained by estimating a primary ray image to be obtained by imaging the virtual model K is combined with an estimated scattered ray image obtained by estimating a scattered ray image to be obtained by imaging the virtual model K, as an estimated image obtained by estimating the first radiation image G1 obtained by imaging the subject H.

Next, the scattered ray removal unit 22 corrects the initial body thickness distribution TO(x,y) of the virtual model K such that a difference between the estimated image and the first radiation image G1 is small. The scattered ray removal unit 22 repeatedly performs the generation of the estimated image and the correction of the body thickness distribution until the difference between the estimated image and the first radiation image G1 satisfies a predetermined end condition. The scattered ray removal unit 22 derives the body thickness distribution in a case where the end condition is satisfied, as the body thickness distribution T(x,y) of the subject H. Further, the scattered ray removal unit 22 subtracts the scattered ray component in a case where the end condition is satisfied from the first radiation image G1 to remove the scattered ray component included in the first radiation image G1. In the following description, it is assumed that the scattered ray components are removed from the first and second radiation images G1 and G2.

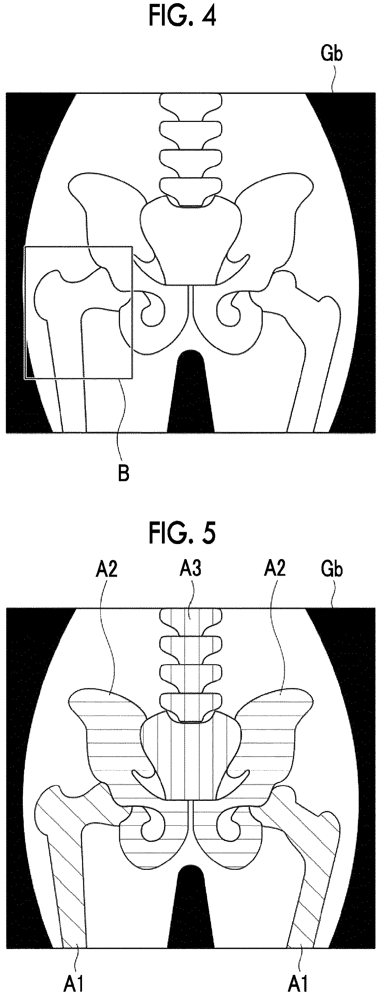

The bone part image derivation unit 23 performs energy subtraction processing to derive a bone part image Gb in which a bone part of the subject H is extracted from the first and second radiation images G1 and G2. When the bone part image Gb is derived, the bone part image derivation unit 23 performs weighted subtraction on the first and second radiation images G1 and G2 between respectively corresponding pixels, as shown in Equation (1), to derive the bone part image Gb in which the bone part of the subject H included in each of the radiation images G1 and G2 is extracted, as shown in Fig. 4. In Equation (1), $\beta 1$ is a weighting coefficient. In Fig. 4, a partial image B described below is shown.

$$Gb(x,y) = G1(x,y) - \beta 1 \times G2(x,y) \ ... \ (1)$$

The segmentation unit 24 segments the bone part image Gb into a femoral region, a pelvis region, and a vertebral region. The segmentation may be performed by using an extraction model in which machine learning is performed to respectively extract the femur, the pelvis, and the vertebra from the bone part image Gb. Further, templates respectively representing the femur, the pelvis, and the vertebra may be stored in the storage 13, and template matching between these templates and the bone part image Gb may be performed to perform the segmentation.

Fig. 5 is a diagram showing a result of the segmentation by the segmentation unit 24. As shown in Fig. 5, the bone region in the bone part image Gb is segmented into a femoral region A1, a pelvis region A2, and a vertebral region A3. In Fig. 5, the result of the segmentation is shown by applying different hatching to the femoral region A1, the pelvis region A2, and the vertebral region A3.

On the other hand, regarding the vertebra, the bone part image Gb includes only a sacral vertebra and a lumbar vertebra. The lumbar vertebrae are anatomically classified from the pelvis to the neck into fifth to first lumbar vertebrae L5, L4, L3, L2, and L1. Therefore, it is preferable that the segmentation unit 24 segments the sacral vertebra and the five lumbar vertebras into different regions.

Note that the segmentation unit 24 may segment only the bone of interest in the bone part image Gb. For example, since the femur is used as the bone of interest in the present embodiment, only the femoral region A1 may be segmented. In addition, the segmentation unit 24 may segment the first radiation image G1 or the second radiation image G2 instead of the bone part image Gb.

The likelihood derivation unit 25 derives the likelihood of osteoporosis based on the bone part image Gb or the partial image including the bone of interest in the bone part image Gb. In a case of deriving the likelihood based on the partial image, the likelihood derivation unit 25 extracts the partial image from the bone part image Gb. The partial image B is an image including a femur, which is a bone of interest extracted by the segmentation unit 24, and a region around the femur in the bone part image Gb, as shown in Fig. 4, for example. The partial image may be only an image of a region of the femur, which is the bone of interest. In addition, the likelihood derivation unit 25 may derive the centroid of the femur and extract a predetermined range in which the centroid of the femur is the centroid, as the partial image.

In some cases, the bone of interest is a vertebra. In this case, the likelihood derivation unit 25 derives the likelihood of osteoporosis based on the partial image including the vertebra. Therefore, the likelihood derivation unit 25 extracts a partial image including the vertebra as the bone of interest. In this case, the centroid of the vertebra may be derived, and the partial image may be derived such that the centroid is the centroid of the partial image. In addition, a centroid position of each of a plurality of vertebrae (for example, a second lumbar vertebra, a third lumbar vertebra, and a fourth lumbar vertebra) may be derived, and the partial image may be derived such that the derived centroid position of the partial image is the centroid of the partial image.

In the present embodiment, in a case where the partial image B is input, the likelihood derivation unit 25 derives the likelihood P0 of osteoporosis by using the trained model 25A that outputs the likelihood P0 of osteoporosis.

The trained model 25A is constructed by subjecting the neural network to machine learning using, as teacher data, a partial image including the bone of interest in the bone part image Gb and correct answer data representing the presence or absence of the onset of osteoporosis with respect to the bone part image Gb. Here, in a case where osteoporosis is developed, the density of the trabecular structure of the cancellous bone included in the bone part is decreased. Therefore, the correct answer data of the teacher data is obtained by determining whether or not osteoporosis is developed after the doctor determines the situation of the trabecular structure by interpreting the bone part image Gb.

Examples of the neural network include a simple perceptron, a multilayer perceptron, a deep neural network, a convolutional neural network, a deep belief network, a recurrent neural network, and a probabilistic neural network. In the present embodiment, the convolutional neural network is used as the neural network.

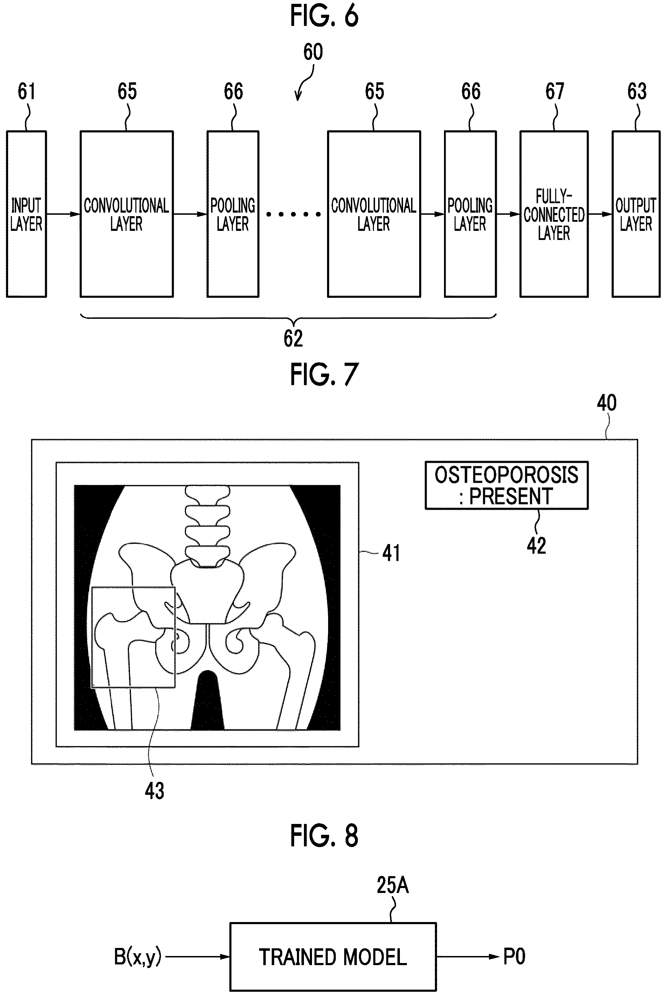

Fig. 6 is a diagram showing the neural network used in the present embodiment. As shown in Fig. 6, a neural network 60 comprises an input layer 61, an interlayer 62, and an output layer 63. The interlayer 62 comprises, for example, a plurality of convolutional layers 65, a plurality of pooling layers 66, and a fully connected layer 67. In the neural network 60, the fully connected layer 67 is present in a previous stage of the output layer 63. In the neural network 60, the convolutional layer 65 and the pooling layer 66 are alternately disposed between the input layer 61 and the fully connected layer 67.

Note that a configuration of the neural network 60 is not limited to the example of Fig. 6. For example, the neural network 60 may comprise one convolutional layer 65 and one pooling layer 66 between the input layer 61 and the fully connected layer 67.

Fig. 7 is a diagram showing an example of teacher data used for training the neural network in the first embodiment. As shown in Fig. 7, teacher data 40 consists of training data 41 and correct answer data 42. The training data 41 consists of a partial image 43 including the bone of interest in the bone part image. In Fig. 7, the partial image includes the femur, which is the bone of interest, and the surrounding region. The partial image 43 is derived from a bone part image of a healthy person and a bone part image of a patient who has developed osteoporosis.

The presence or absence of osteoporosis as the correct answer data 42 is set according to whether the partial image 43 is derived from the bone part image of the healthy person or the bone part image of the patient with osteoporosis.

The correct answer data 42 consists of the presence or absence of osteoporosis. In the present embodiment, the likelihood P0 is assumed to have a value of 0 to 1. Therefore, the value of the correct answer data 42 in a case of having osteoporosis is 1, and the value of the correct answer data 42 in a case of not having osteoporosis is 0.

The partial image 43, which is the training data 41, may be derived by processing a bone part image of a healthy person according to the presence or absence of osteoporosis. Accordingly, it is possible to increase the number of pieces of teacher data and thus effectively promote the learning.

In a case where the trained model 25A, which was constructed by the machine learning of a neural network with a large number of such teacher data sets 40, receives the pixel values B(x, y) of a partial image B including the bone of interest within the bone part image Gb of the subject H, who is a patient, as shown in Fig. 8, the likelihood P0 that the patient H has osteoporosis is output.

in a case where the likelihood derivation unit 25 derives the likelihood P0 of osteoporosis based on the bone part image Gb instead of the partial image, the trained model 25A is constructed to output the likelihood P0 of osteoporosis in a case where the bone part image Gb is input. The training data 41 of the teacher data 40 used in this case is a bone part image of a healthy person and a patient with osteoporosis.

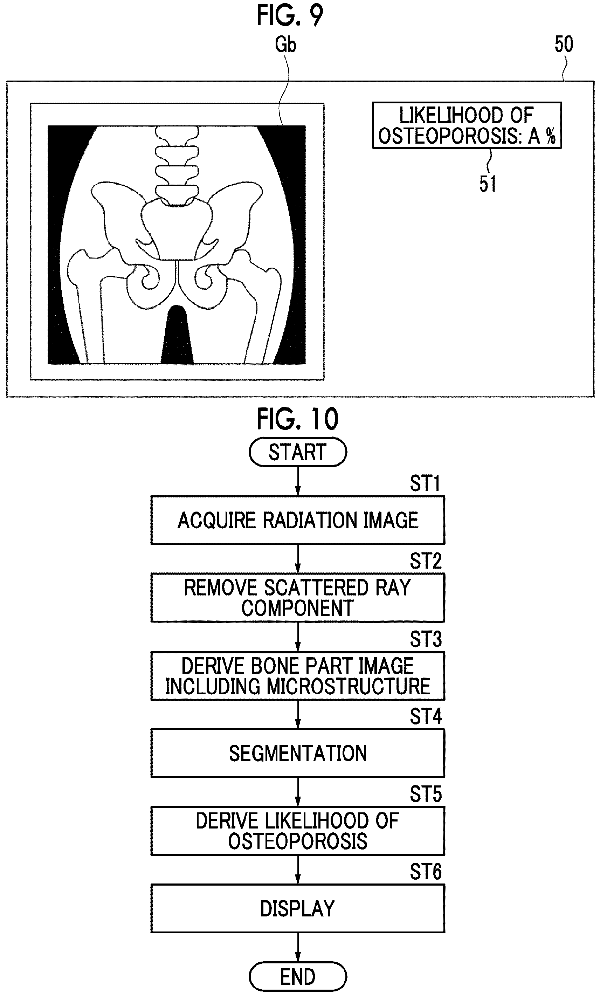

The display control unit 26 displays the likelihood P0 on the display 14 in order to issue a notification of the likelihood P0 of osteoporosis derived by the likelihood derivation unit 25. Fig. 9 is a diagram showing a display screen of the likelihood of osteoporosis. As shown in Fig. 9, the display screen 50 displays the bone part image Gb of the subject H and the likelihood 51 of osteoporosis. The likelihood takes a value of 0 to 1, but in the display screen 50 shown in Fig. 9, the likelihood is indicated by a percentage.

Next, a processing performed in the first embodiment will be described. Fig. 10 is a flowchart showing a process performed in the first embodiment. Note that the first and second radiation images G1 and G2 are acquired by the imaging and stored in the storage 13. In a case where an instruction for starting the processing is input from the input device 15, the image acquisition unit 21 acquires the first and second radiation images G1 and G2 from the storage 13 (radiation image acquisition; step ST1).

Then, the scattered ray removal unit 22 removes the scattered ray components from the first and second radiation images G1 and G2 (step ST2). In addition, the bone part image derivation unit 23 derives a bone part image Gb including a microstructure inside the bone part of the subject H from the first and second radiation images G1 and G2 (step ST3). Further, the segmentation unit 24 segments a region of the femur, which is the bone of interest, in the bone part image Gb (step ST4).

Subsequently, the likelihood derivation unit 25 extracts the partial image B including the bone of interest in the bone part image Gb, and derives the likelihood P0 of osteoporosis based on the partial image B (Step ST5). Then, the display control unit 26 displays the likelihood P0 of osteoporosis derived by the likelihood derivation unit 25 on the display 14 (Step ST6), and the processing is ended.

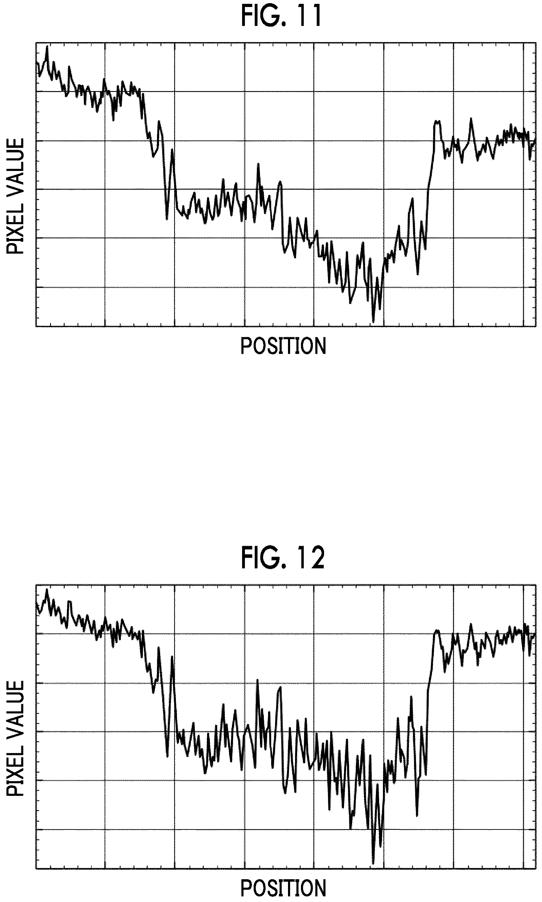

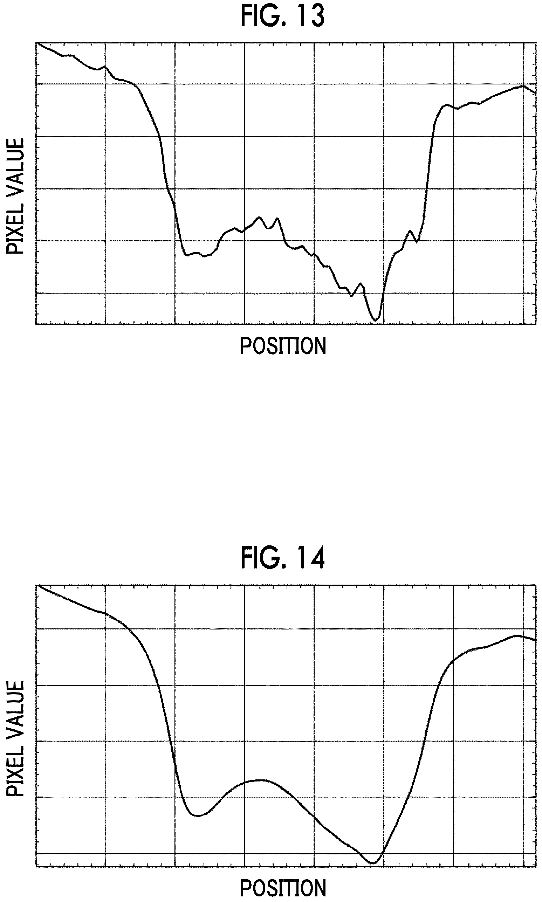

Figs. 11 to 14 are diagrams for describing spatial resolution according to the type of the image. Fig. 11 is a graph showing a profile of a radiation image acquired in the present embodiment, Fig. 12 is a graph showing a profile of a bone part image derived from two radiation images acquired in the present embodiment, Fig. 13 is a graph showing a profile of a bone part image corresponding to a bone density image acquired by the quantitative CT method, and Fig. 14 is a graph showing a profile of a bone part image corresponding to a bone density image acquired by the DXA method.

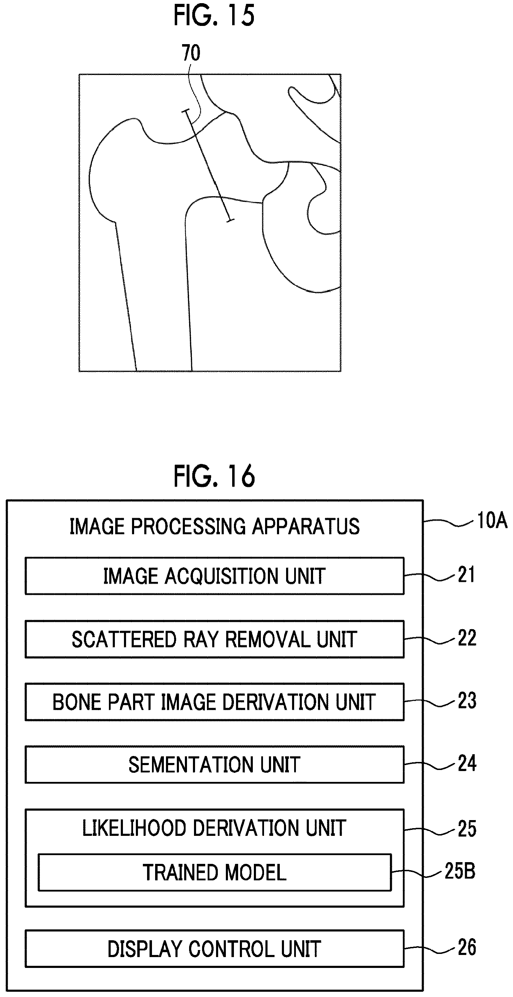

In addition, the horizontal axis of the graphs shown in Figs. 11 to 14 indicates a position in a section 70 in the neck portion of the femur as shown in Fig. 15, and the vertical axis indicates a pixel value in the section 70. In addition, the interval of the vertical lines is 10 mm. In addition, the spatial resolution of the radiation image and the bone part image are 0.15 mm/pixel, the spatial resolution of the bone part image corresponding to the bone density image acquired by the quantitative CT method is 1.0 mm/pixel, and the spatial resolution of the bone part image corresponding to the bone density image acquired by the DXA method is 2.0 mm/pixel.

As shown in the graph of Fig. 11, in the radiation image acquired in the present embodiment, it can be seen that the trabecular structure of the cancellous bone is depicted since the pixel value varies finely inside the bone in the section 70. In addition, as shown in the graph of Fig. 12, in the bone part image acquired in the present embodiment, the pixel value varies finely inside the bone in the section 70, and the width of the variation is larger than that in Fig. 11. Therefore, it can be seen that in the bone part image acquired in the present embodiment, the trabecular structure of the cancellous bone is more clearly depicted than in the radiation image.

On the other hand, as shown in the graph of Fig. 13, in the bone part image corresponding to the bone density image acquired by the quantitative CT method, it can be seen that the trabecular structure of the cancellous bone is hardly depicted since there is hardly any fine fluctuation in the pixel value inside the bone in the section 70. In addition, as shown in the graph of Fig. 14, in the bone part image corresponding to the bone density image acquired by the DXA method, it can be seen that the trabecular structure of the cancellous bone is not depicted at all since there is no fine fluctuation in the pixel value inside the bone in the section 70.

In the first embodiment, the bone part image Gb including the microstructure inside the bone part is derived, and the likelihood P0 of osteoporosis is derived based on the partial image B including the bone of interest in the bone part image Gb. That is, as shown in the graph of Fig. 12, the likelihood P0 of osteoporosis is derived from the bone part image Gb in which the fine trabecular structure of the cancellous bone inside the bone part is clearly depicted. Here, the first and second radiation images G1 and G2 for acquiring the bone part image Gb are acquired by capturing simple radiation images. Therefore, according to the present embodiment, it is possible to simply and accurately determine osteoporosis.

In addition, since the scattered ray components are removed from the first and second radiation images G1 and G2, it is possible to derive the bone part image Gb in which the blurriness of the image due to the scattered rays is small. Therefore, it is possible to derive the likelihood P0 of osteoporosis with higher accuracy.

In this case, the scattered ray removal unit 22 is not necessary.

Next, a second embodiment of the present disclosure will be described. Fig. 16 is a diagram showing a functional configuration of the image processing apparatus according to the second embodiment. In the first embodiment, the likelihood derivation unit 25 employs a trained model 25A, obtained by machine learning, that outputs the likelihood P0 of osteoporosis in a case where supplied with either the bone part image Gb or a partial image including the bone of interest within the bone part image Gb. The image processing apparatus 10A according to the second embodiment differs from the first embodiment in that, in a case where the imaging conditions when the first and second radiation images G1 and G2 are acquired are input in addition to the bone part image Gb or the partial image B including the bone of interest in the bone part image Gb, a trained model 25B in which the machine learning is performed to output the likelihood P0 of osteoporosis is used.

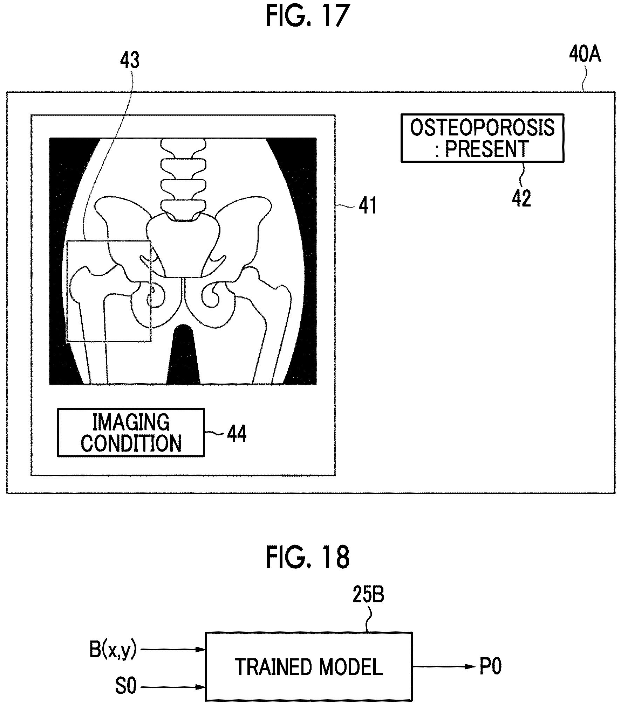

Fig. 17 is a diagram showing an example of teacher data used for learning the neural network in the second embodiment. As shown in Fig. 17, the teacher data 40A used for the learning of the neural network in the second embodiment includes the imaging condition 44 in a case where the training data 41 is acquired, in addition to the teacher data 40 shown in Fig. 7.

In a case where the trained model 25B, which is constructed by the machine learning of a neural network with a large number of such teacher data sets 40A, is supplied with the pixel values B(x, y) per pixel in either the bone part image Gb of the subject H, who is a patient, or a partial image including the bone of interest within the bone part image Gb, and the imaging condition S0 under which the first and second radiographic images G1 and G2 used to generate the bone-part image Gb were acquired, the model, as shown in Fig. 18, outputs the likelihood P0 that the subject H has osteoporosis.

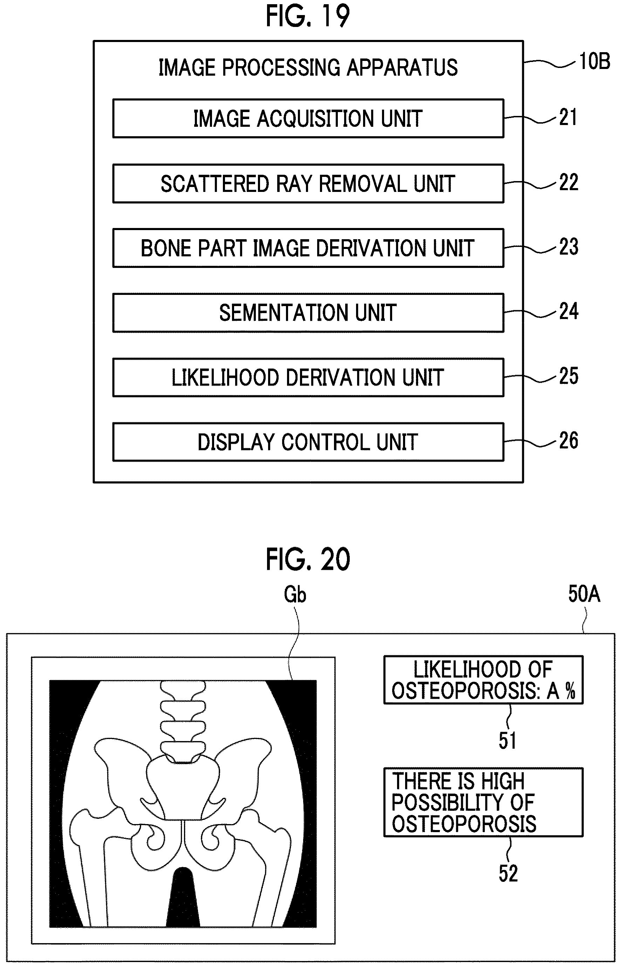

Next, description regarding a third embodiment of the present disclosure will be made. Fig. 19 is a diagram showing a functional configuration of the image processing apparatus according to the third embodiment. In the first and second embodiments, the likelihood derivation unit 25 derives the likelihood P0 of osteoporosis using the trained models 25A and 25B. The image processing apparatus 10B according to the third embodiment is different from the first and second embodiments in that the likelihood derivation unit 25 derives the likelihood P0 of osteoporosis based on the statistical value of the partial image including the bone of interest in the bone part image Gb.

In the third embodiment, the likelihood derivation unit 25 derives a statistical value of the bone part image Gb or a partial image B including the bone of interest in the bone part image Gb. For example, the likelihood derivation unit 25 derives, as the statistical value, at least one of an average value $\mu A$, a variance $\sigma A$, a maximum value MaxA, and a minimum value MinA of pixel values of only the region of the bone of interest included in the partial image B including the region of the bone of interest and the region around the region of the bone of interest, and an average value $\mu B$, a variance $\sigma B$, a maximum value MaxB, and a minimum value MinB of only the region around the region of the bone of interest.

Here, in a case where osteoporosis is developed, the density of the trabecular structure of the cancellous bone is reduced, and thus the average values $\mu A$ and $\mu B$ are reduced because the number of high-brightness (low-concentration) components is reduced. In addition, in a case where the density of the trabecular structure of the cancellous bone is reduced, a difference in sparse-and-dense patterns of the trabeculae, that is, a difference in pixel values is increased, so that the variances $\sigma A$ and $\sigma B$ are reduced. In addition, in a case where the density of the trabecular structure of the cancellous bone is reduced, the maximum values MaxA and MaxB and the minimum values MinA and MinB are reduced because the number of high-brightness (low-concentration) components is reduced.

The likelihood derivation unit 25 may derive the likelihood P0 of osteoporosis by normalizing any one of the eight statistical values to 0 to 1, but in the third embodiment, the likelihood P0 is derived by performing a weighting operation on the eight statistical values according to Equation (2). In Equation (2), C1 to C8 are weighting coefficients for each of the eight statistical values. In addition, among the weighting coefficients C1 to C8, the weighting coefficients C1 and C2 with respect to the average value $\mu A$ and the variance $\sigma A$ of the pixel values of only the region of the bone of interest are set to values larger than the other weighting coefficients C3 to C8.

$$P0 = \frac{C2 \times \sigma A + C6 \times \sigma B}{C1 \times \mu A + C3 \times Max A + C4 \times Min A + C5 \times \mu B + C7 \times Max B + C8 \times Min B} \quad (2)$$

The weighting coefficients C1 to C8 in Equation (2) can be obtained by machine learning a discriminator such as AdaBoost or SVM. Alternatively, a method of discriminant analysis using a Mahalanobis distance can be used. For example, in a case where AdaBoost is used, the above-described statistical values may be derived for a large number of images of osteoporosis and a large number of normal images, and the weighting coefficient multiplied by each statistical value may be optimized such that the discrimination accuracy of osteoporosis is highest (that is, the error rate is minimized).

Even in the third embodiment, as shown in the graph of Fig. 12, the fine trabecular structure of the cancellous bone inside the bone part is clearly depicted, and the likelihood P0 of osteoporosis is derived from the bone part image Gb derived from the first and second radiation images G1 and G2 acquired by the simple imaging. Therefore, according to the third embodiment, similarly to the first embodiment, it is possible to simply and accurately determine osteoporosis.

In each of the above-described embodiments, as shown in Fig. 9, the display screen 50 including the bone part image Gb and the likelihood 51 of osteoporosis is displayed on the display 14, but the present disclosure is not limited thereto. From the progression of osteoporosis, a warning may be issued in a case where the likelihood P0 derived by the likelihood derivation unit 25 is equal to or greater than a predetermined threshold value. The comparison between the likelihood P0 and the threshold value may be performed by the likelihood derivation unit 25, may be performed by the display control unit 26, or a function dedicated to the comparison may be provided in the image processing apparatus 10.

Fig. 20 is a diagram showing a display screen including a warning. As shown in Fig. 20, the display screen 50A includes a warning display 52 in addition to the bone part image Gb and the likelihood 51 of osteoporosis. The warning display 52 includes a text "there is a high possibility of osteoporosis".

In this way, by issuing the warning in a case where the likelihood P0 is equal to or greater than the predetermined threshold value, the doctor can easily recognize that the patient from which the bone part image Gb is derived has a high possibility of osteoporosis.

In addition, the priority of the treatment for osteoporosis may be determined based on the likelihood P0 of osteoporosis derived by the likelihood derivation unit 25, and the priority of the treatment may be displayed in addition to the bone part image Gb and the likelihood 51 of osteoporosis. The determination of the priority of the treatment may be performed by the likelihood derivation unit 25, may be performed by the display control unit 26, or a function dedicated to the determination of the priority of the treatment may be provided in the image processing apparatus 10.

Here, in the present embodiment, the likelihood P0 of osteoporosis is a value of 0 to 1. Therefore, the priority need only be decided as "high" in a case where the likelihood P0 is 0.7 to 1.0, "medium" in a case where the likelihood P0 is 0.3 to 0.7, and "low" in a case where the likelihood P0 is 0.0 to 0.3.

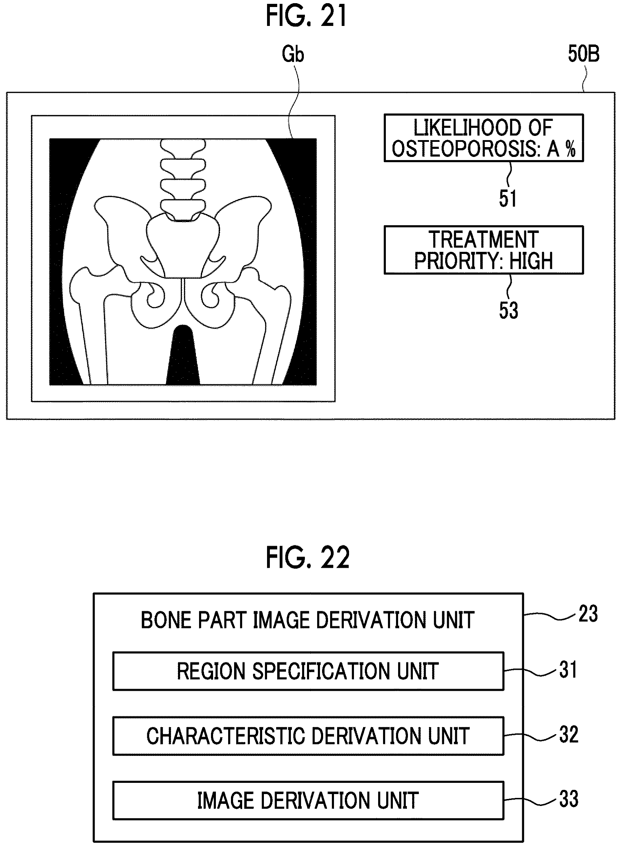

Fig. 21 is a diagram showing a display screen including a priority of treatment. As shown in Fig. 21, a display screen 50B includes the bone part image Gb, the likelihood 51 of osteoporosis, and a priority 53 of the treatment. In Fig. 21, the priority 53 of the treatment is "high".

In this way, it is possible to use the determined priority for triage of the patient by determining the priority of the treatment based on the likelihood P0 of osteoporosis and notifying the determined priority.

In addition, in each of the above-described embodiments, the bone part image derivation unit 23 derives the bone part image Gb by performing the weighted subtraction on the first radiation image G1 and the second radiation image G2 by Equation (1), but the present disclosure is not limited to this. The bone part image Gb may be derived using the characteristics related to the attenuation of the radiation in the bone part and the soft part of the subject H. This will be described below as a fourth embodiment.

Fig. 22 is a diagram showing a functional configuration of a bone part image derivation unit in the fourth embodiment. As shown in Fig. 22, in the fourth embodiment, the bone part image derivation unit 23 comprises a region specification unit 31, a characteristic derivation unit 32, and an image derivation unit 33.

The first and second radiation images G1 and G2 acquired by the image acquisition unit 21 include a region of the subject H and a direct radiation region obtained by directly irradiating the radiation detectors 5 and 6 with radiation. A soft region and a bone region are included in the region of the subject H. A soft part component of a human body includes muscle, fat, blood, and water. In the present embodiment, a non-fat tissue including blood and water is treated as the muscle.

The soft regions of the first and second radiation images G1 and G2 include only the soft part component of the subject H. The bone regions of the first and second radiation images G1 and G2 are actually regions in which the bone part component and the soft part component are mixed.

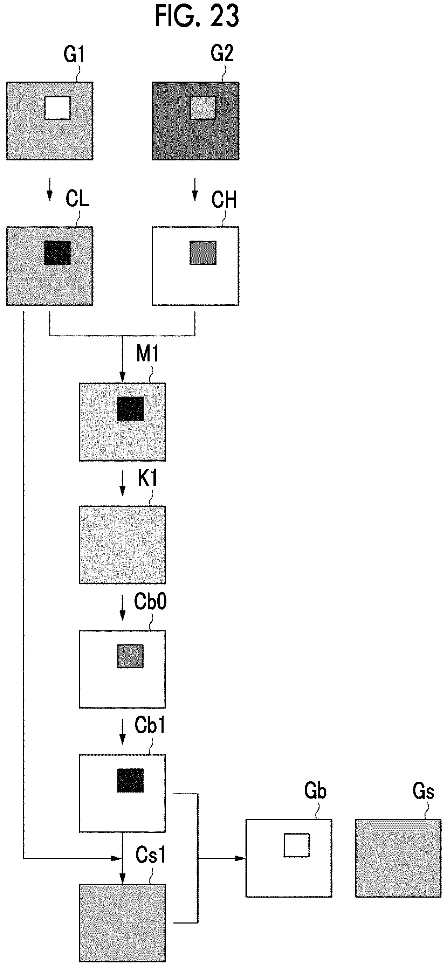

Hereinafter, processing performed by the region specifying unit 31, the characteristic derivation unit 32, and the image derivation unit 33 will be described. Fig. 23 is a diagram schematically showing processing of deriving a bone part image according to the fourth embodiment. Note that, in Fig. 23, in order to simplify the description, the first radiation image G1 and the second radiation image G2 do not include the direct radiation region, and include a rectangular bone region in the soft region.

The region specifying unit 31 specifies a bone region and a soft region in the first radiation image G1 or the second radiation image G2. For this reason, the region specifying unit 31 derives an attenuation characteristic related to the attenuation of the radiation in at least the region of the subject H of the first radiation image G1 or the second radiation image G2, and specifies the soft region and the bone region based on the attenuation characteristic in the region of the subject H. The region specifying unit 31 derives a first attenuation image CL and a second attenuation image CH representing an attenuation amount of the radiation by the subject H from each of the first radiation image G1 and the second radiation image G2, and derives an attenuation ratio, which is a ratio between the corresponding pixels of the first attenuation image CL and the second attenuation image CH, as the attenuation characteristic.

Here, a pixel value of the first attenuation image CL represents the attenuation amount of the low-energy radiation due to the subject H, and a pixel value of the second attenuation image CH represents the attenuation amount of the high-energy radiation due to the subject H. The first attenuation image CL and the second attenuation image CH are derived from the first radiation image G1 and the second radiation image G2 by Equation (3) and Equation (4). In the Equation (3), Gd1 is the pixel value of the direct radiation region in the first radiation image G1, and, in the Equation (4), Gd2 is the pixel value of the direct radiation region in the second radiation image G2.

$$CL(x,y) = Gd1 - G1(x,y) ... (3)$$

$$CH(x,y) = Gd2 - G2(x,y) ... (4)$$

Next, the region specifying unit 31 derives an attenuation ratio map representing the attenuation ratio of the radiation between the first radiation image G1 and the second radiation image G2. Specifically, an attenuation ratio map M1 is derived by deriving a ratio between the corresponding pixels of the first attenuation image CL and the second attenuation image CH by Equation (5). The attenuation ratio is an example of a characteristic related to the attenuation of the radiation.

$$M1(x,y) = CL(x,y) / CH(x,y) ... (5)$$

Here, in the first radiation image G1 and the second radiation image G2, the attenuation ratio of the region including only the soft part component is smaller than the attenuation ratio of the region including the bone part component. Therefore, the region specifying unit 31 compares the attenuation ratios of the respective pixels of the attenuation ratio map M1, specifies a region consisting of the pixel in which the attenuation ratio is larger than a predetermined threshold value as the bone region, and specifies a region other than the bone region as the soft region. Note that the region specifying unit 31 may compare the attenuation ratio between each pixel of the attenuation ratio map M1 with the surrounding pixels, and may specify the pixel having a larger attenuation ratio than surrounding pixels as the pixel in the bone region.

The characteristic derivation unit 32 derives the characteristics of the soft part related to the attenuation of the radiation based on the first radiation image G2 and the second radiation image G2 in the soft region including only the soft part of the first radiation image G1 or the second radiation image G2. In addition, the characteristic derivation unit 32 derives the characteristics of the soft part in the bone region based on the characteristics of the soft part derived in the soft region around the bone region. In the present embodiment, the characteristic derivation unit 32 derives the attenuation ratio between the first radiation image G1 and the second radiation image G2 as the characteristic of the soft part.

Here, in the fourth embodiment, the region specifying unit 31 derives the attenuation ratio, which is the characteristic related to the attenuation of the radiation, in the region of the subject H in the first radiation image G1 or the second radiation image G2. Therefore, the characteristic derivation unit 32 uses the attenuation ratio of the soft region in the attenuation ratio map M1 derived by the region specifying unit 31 as the characteristic of the soft part in the soft region. That is, the region specification unit 31 also has a function as the characteristic derivation unit 32.

On the other hand, for the bone region, the attenuation ratio of the soft region around the bone region is interpolated to derive the characteristic of the soft part for the bone region, that is, the attenuation ratio. Note that, instead of the interpolation, a median value of the attenuation ratio of the soft region in the attenuation ratio map M1, an average value thereof, or a value thereof that is a predetermined ratio from the small attenuation ratio side may be derived as the attenuation ratio for the bone region.

As a result, the characteristic derivation unit 32 derives the characteristic of the soft part for the region of the subject H of the first radiation image G1 or the second radiation image G2. The characteristics of the soft part are the attenuation ratios of the soft region. In the fourth embodiment, the derived characteristic of the soft part is used as a soft part removal coefficient K1 for removing the soft part in a case where the image derivation unit 33, which will be described below, derives the bone part image.

The image derivation unit 33 derives a bone part image Gb in which the bone part component is emphasized, based on the characteristics of the soft part in the region of the subject H in the first radiation image G1 or the second radiation image G2.

In the fourth embodiment, the image derivation unit 33 first derives an initial bone part attenuation image in which the bone part is emphasized, based on the first attenuation image CL, the second attenuation image CH, and the soft part removal coefficient K1 which is the characteristic of the soft part. Specifically, the image derivation unit 33 derives an initial bone part attenuation image Cb0 by Equation (6).

$$Cb0(x,y) = CL(x,y) - CH(x,y) \times K1(x,y) ... (6)$$

Here, as the pixel value of the bone region of the initial bone part attenuation image Cb0 derived as described above, there are the pixel value obtained by replacing the attenuation amount of the bone part with the attenuation amount of the soft part by assuming that the soft part corresponding to the thickness of the bone part is present, and the pixel value representing a difference from an actual attenuation amount of the bone part. Therefore, a contrast is low with respect to the bone part attenuation image that is originally desired to be derived. In a case where the bone part attenuation image having such a low contrast is used, in a case where a soft part attenuation image is derived by subtracting the bone part attenuation image from the first attenuation image CL or the second attenuation image CH as will be described below, the bone part component cannot be removed satisfactorily.

Therefore, the image derivation unit 33 derives the bone part attenuation image Cb1 by matching the contrast of the initial bone part attenuation image Cb0 with the contrast of the first attenuation image CL or the second attenuation image CH. In the fourth embodiment, the contrast of the initial bone part attenuation image Cb0 is matched with the contrast of the first attenuation image CL. Therefore, the image derivation unit 33 converts the contrast of the initial bone part attenuation image Cb0 by multiplying the initial bone part attenuation image Cb0 by a contrast conversion coefficient. Then, a correlation between a difference image $\Delta$CL derived by subtracting the initial bone part attenuation image Cb0 after the contrast conversion from the first attenuation image CL and the initial bone part attenuation image Cb0 is derived. Then, the contrast conversion coefficient is determined so that the correlation is minimized, and the determined contrast conversion coefficient is multiplied by the initial bone part attenuation image Cb0 to derive the bone part attenuation image Cb1.

Note that a table representing the contrast conversion coefficient in which the contrast of the initial bone part attenuation image Cb0 and a body thickness are associated with each other may be created in advance. In this case, the bone part attenuation image Cb1 may be derived by deriving the body thickness of the subject H by the measurement or the like, deriving the contrast conversion coefficient with reference to the table from the contrast and the body thickness of the initial bone part attenuation image Cb0, and converting the initial bone part attenuation image Cb0 by using the derived contrast conversion coefficient.

Then, the image derivation unit 33 derives a soft part attenuation image Cs1 by subtracting the bone part attenuation image Cb1 from the first attenuation image CL by Equation (7).

$$Cs1(x,y) = CL(x,y) - Cb1(x,y) \dots (7)$$

Further, the image derivation unit 33 derives the bone part image Gb by Equation (8). In a case of deriving a soft part image Gs, the image derivation unit 33 derives the soft part image Gs by Equation (9).

$$Gb(x,y) = Gd1(x,y) - Cb1(x,y) \dots (8)$$

$$Gs(x,y) = Gd2(x,y) - Cs1(x,y) \dots (9)$$

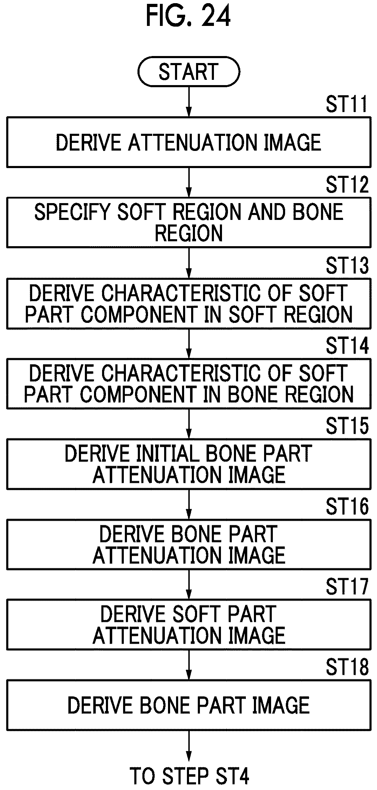

Next, processing of deriving a bone part image performed in the fourth embodiment will be described. Fig. 24 is a flowchart showing bone part image derivation processing performed in the fourth embodiment. It is assumed that the processing of acquiring the first and second radiation images G1 and G2 by the image acquisition unit 21, and of removing the scattered ray components from the first and second radiation images G1 and G2 by the scattered ray removal unit 22 have been completed. Therefore, in the following description, it is assumed that the scattered ray components are removed from the first and second radiation images G1 and G2.

First, the region specifying unit 31 derives the first attenuation image CL and the second attenuation image CH from the first radiation image G1 and the second radiation image G2 (attenuation image derivation: step ST11), and specifies the soft region including only the soft part component and the bone region including the bone part in the first attenuation image CL or the second attenuation image CH (step ST12). Next, the characteristic derivation unit 32 derives the characteristic (attenuation ratio) of the soft part component related to the attenuation of the radiation image in the soft region (step ST13). Then, the characteristic derivation unit 32 derives the characteristic of the soft part component in the bone region (step ST14).

Next, the image derivation unit 33 derives the initial bone part attenuation image Cb0 (step ST15), and derives the bone part attenuation image Cb1 by converting the contrast of the initial bone part attenuation image Cb0 (step ST16). Further, the image derivation unit 33 derives the soft part attenuation image Cs1 by subtracting the bone part attenuation image Cb1 from the first attenuation image CL (step ST17). Subsequently, the image derivation unit 33 derives the bone part image Gb by Equation (8) (Step ST18). After the derivation of the bone part image Gb, the process proceeds to the processing of step ST4 of the flowchart shown in Fig. 10 described above.

In each of the above-described embodiments, the femur is used as the bone of interest, but the present disclosure is not limited thereto. The vertebra may be set as the region of interest.

Particularly, the bone mineral density of the vertebra is reduced due to occurrence of osteoporosis. In a case where osteoporosis worsens, the vertebra is compressed and deformed in the vertical direction of the human body, and further is compression fractured. Therefore, even in a case where the bone of interest is the vertebra, the likelihood P0 of osteoporosis can be derived according to the present embodiment.

Further, in the present embodiment, in addition to the femur and the vertebra, any bone such as the femur and a shinbone around a knee joint can be used as the bone of interest.

In addition, in each of the above-described embodiments, the scattered ray components are removed from the first and second radiation images G1 and G2, but the present disclosure is not limited thereto. The bone part image Gb may be derived without removing the scattered ray components from the first and second radiation images G1 and G2.

Further, in each embodiment described above, the first and second radiation images G1 and G2 are acquired by the one-shot method in a case where the energy subtraction processing is performed, but the present disclosure is not limited thereto. The first and second radiation images G1 and G2 may be acquired by a so-called two-shot method in which imaging is performed twice by using only one radiation detector. In a case of the two-shot method, there is a possibility that a position of the subject H included in the first radiation image G1 and the second radiation image G2 deviates due to a body movement of the subject H. Therefore, in the first radiation image G1 and the second radiation image G2, it is preferable to perform the processing according to the present embodiment after registration of the subject is performed.

Further, in each of the embodiments described above, the image processing is performed by using the radiation image acquired by the system that images the first and second radiation images G1 and G2 of the subject H by using the first and second radiation detectors 5 and 6, it is needless to say that the technology of the present disclosure can be applied to even in a case where the first and second radiation images G1 and G2 are acquired by using an accumulative phosphor sheet instead of the radiation detector. In this case, the first and second radiation images G1 and G2 need only be acquired by stacking two accumulative phosphor sheets, emitting the radiation transmitted through the subject H, accumulating and recording radiation image information of the subject H in each of the accumulative phosphor sheets, and photoelectrically reading the radiation image information from each of the accumulative phosphor sheets. Note that the two-shot method may also be used in a case where the first and second radiation images G1 and G2 are acquired by using the accumulative phosphor sheet.

Further, the radiation in the embodiments described above is not particularly limited, and $\alpha$-rays or $\gamma$-rays can be used in addition to X-rays.

In addition, in the above-described embodiment, for example, as a hardware structure of processing units that execute various processing, such as the image acquisition unit 21, the scattered ray removal unit 22, the bone part image derivation unit 23, the segmentation unit 24, the likelihood derivation unit 25, the display controller 26, the region specifying unit 31, the characteristic derivation unit 32, and the image derivation unit 33, various processors shown below can be used. The various processors include a programmable logic device (PLD) which is a processor whose circuit configuration is changeable after manufacturing such as a field programmable gate array (FPGA), a dedicated electric circuit which is a processor having a circuit configuration exclusively designed to execute specific processing such as an application specific integrated circuit (ASIC), and the like, in addition to the CPU which is a general-purpose processor that executes software (program) to function as various processing units, as described above.

One processing unit may be configured by one of the various processors, or may be configured by a combination of the same or different types of two or more processors (for example, a combination of a plurality of FPGAs or a combination of the CPU and the FPGA). Further, a plurality of processing units may be composed of one processor.

As an example where a plurality of processing units are configured by one processor, first, there is a form in which one processor is configured by a combination of one or more CPUs and software as typified by a computer, such as a client or a server, and this processor functions as a plurality of processing units. Second, as represented by a system-on-chip (SoC) or the like, there is a form of using a processor for realizing the function of the entire system including a plurality of processing units with one integrated circuit (IC) chip. In this way, various processing units are configured by one or more of the above-described various processors as hardware structures.

Furthermore, as the hardware structure of the various processors, more specifically, an electrical circuit (circuitry) in which circuit elements such as semiconductor elements are combined can be used.

The supplementary notes of the present disclosure will be described below.

(Supplementary Note 1)

An image processing apparatus comprising at least one processor, in which the processor is configured to:

acquire two radiation images acquired by imaging a subject including a bone part and a soft part with radiation having different energy distributions;
derive a bone part image including a microstructure inside the bone part based on the two radiation images; and
derive a likelihood of osteoporosis based on the bone part image or a partial image including a bone of interest in the bone part image.

(Supplementary Note 2)

The image processing apparatus according to Supplementary Note 1,
in which the bone part image includes a trabecular structure of a cancellous bone included in the bone part.

(Supplementary Note 3)

The image processing apparatus according to Supplementary Note 2,
in which the trabecular structure of the cancellous bone is depicted in the bone part image.

(Supplementary Note 4)

The image processing apparatus according to Supplementary Note 3,
in which the bone part image has a spatial resolution of 0.2 mm/pixel or less.

(Supplementary Note 5)

The image processing apparatus according to any one of Supplementary Notes 1 to 4,
in which the processor is configured to extract the partial image from at least one of the bone part image or the two radiation images.

(Supplementary Note 6)

The image processing apparatus according to Supplementary Note 5,
in which the processor is configured to extract at least one bone of interest from at least one of the bone part image or the two radiation images, and extract the partial image based on a centroid of the bone of interest.

(Supplementary Note 7)

The image processing apparatus according to any one of Supplementary Notes 1 to 6,
in which the processor is configured to function as a trained model in which the machine learning is performed to output a likelihood of osteoporosis in response to an input of the bone part image or the partial image.

(Supplementary Note 8)

The image processing apparatus according to any one of supplementary notes 1 to 6,
in which the processor is configured to function as a trained model that has undergone machine to output the likelihood of osteoporosis in response to an input of imaging conditions of the two radiation images in addition to the bone part image or the partial image.

(Supplementary Note 9)

The image processing apparatus according to any one of Supplementary Notes 1 to 6,
in which the processor is configured to derive the likelihood of osteoporosis based on a statistical value of pixel values of the bone of interest included in the bone part image or the partial image.

(Supplementary Note 10)

The image processing apparatus according to Supplementary Note 9,
in which the statistical value is at least one of an average value, a standard deviation, a maximum value, or a minimum value of pixel values in each of the bone of interest and a peripheral region of the bone of interest.

(Supplementary Note 11)

The image processing apparatus according to Supplementary Note 10,
in which the processor is configured to derive the likelihood by performing a weighting operation on a plurality of statistical values among an average value, a standard deviation, a maximum value, and a minimum value of pixel values in each of the bone of interest and the peripheral region of the bone of interest.

(Supplementary Note 12)

The image processing apparatus according to supplementary note 11,
in which the weight of a weighting operation is derived by machine learning.

(Supplementary Note 13)

The image processing apparatus according to any one of Supplementary Notes 1 to 12,
in which the processor is configured to issue a notification of the likelihood.

(Supplementary Note 14)

The image processing apparatus according to any one of Supplementary Notes 1 to 13,
in which the processor is configured to issue a warning in a case where the likelihood is equal to or greater than a predetermined threshold value.

(Supplementary Note 15)

The image processing apparatus according to any one of Supplementary Notes 1 to 14,

in which the processor is configured to:
determine a priority of a treatment for osteoporosis based on the likelihood; and
issue a notification of the priority.

(Supplementary Note 16)

The image processing apparatus according to any one of Supplementary Notes 1 to 14,
in which the processor is configured to remove scattered ray components included in the two radiation images to derive the bone part image.

(Supplementary Note 17)

The image processing apparatus according to any one of Supplementary Notes 1 to 16,

in which the processor is configured to:
derive, in a soft region including only the soft part of the two radiation images, a characteristic of the soft part related to an attenuation of the radiation based on the two radiation images;
derive, in a bone region including the bone part of the two radiation images, a characteristic of the soft part based on the characteristic of the soft part derived in the soft region around the bone region; and
derive, as the bone part image, an image in which the bone part is emphasized based on the characteristic of the soft part in at least a region of the subject in the two radiation images.

(Supplementary Note 18)

The image processing apparatus according to any one of Supplementary Notes 1 to 17,
in which the bone of interest is a femur or a vertebra.

(Supplementary Note 19)

An image processing method comprising:

via a computer,
acquiring two radiation images acquired by imaging a subject including a bone part and a soft part with radiation having different energy distributions;
deriving a bone part image including a microstructure inside the bone part based on the two radiation images; and
deriving a likelihood of osteoporosis based on the bone part image or a partial image including a bone of interest in the bone part image.

(Supplementary Note 20)

An image processing program causing a computer to execute a process comprising:

acquiring two radiation images acquired by imaging a subject including a bone part and a soft part with radiation having different energy distributions;
deriving a bone part image including a microstructure inside the bone part based on the two radiation images; and
deriving a likelihood of osteoporosis based on the bone part image or a partial image including a bone of interest in the bone part image.

Explanation of References

1: imaging Apparatus
3: radiation source
5, 6: radiation detector
7: radiation energy conversion filter
9: image storage system
10, 10A, 10B: image processing apparatus
11: CPU
12A: image processing program
13: storage
14: display
15: input device
16: memory
17: network I/F
18: bus
21: image acquisition unit
22: scattered ray removal unit
23: bone part image derivation unit
24: segmentation unit
25A, 25B: trained model
26: display control unit
31: region specification section
32: characteristic derivation unit
33: image derivation unit
40, 40A: teacher data
41: training data
42: correct answer data
43: pixel value of partial image including bone of interest
44: imaging condition
50, 50A, 50B: display screen
51: likelihood of osteoporosis
52: warning
53: treatment priority
60: neural network
61: input layer
62: intermediate layer
63: output layer
65: convolutional layer
66: pooling layer
67: fully connected layer
A1: femoral region
A2: pelvis region
A3: vertebral region
B(x,y): bone mineral density per pixel
G1: first radiation image

G2: second radiation image
Gb: bone part image

**Claims**

1. An image processing apparatus comprising at least one processor,
   wherein the processor is configured to:

   acquire two radiation images acquired by imaging a subject including a bone part and a soft part with radiation having different energy distributions;
   derive a bone part image including a microstructure inside the bone part based on the two radiation images; and
   derive a likelihood of osteoporosis based on the bone part image or a partial image including a bone of interest in the bone part image.

2. The image processing apparatus according to claim 1,
   wherein the bone part image includes a trabecular structure of a cancellous bone included in the bone part.

3. The image processing apparatus according to claim 2,
   wherein the trabecular structure of the cancellous bone is depicted in the bone part image.

4. The image processing apparatus according to claim 3,
   wherein the bone part image has a spatial resolution of 0.2 mm/pixel or less.

5. The image processing apparatus according to claim 1,
   wherein the processor is configured to extract the partial image from at least one of the bone part image or the two radiation images.

6. The image processing apparatus according to claim 5,
   wherein the processor is configured to extract at least one bone of interest from at least one of the bone part image or the two radiation images, and extract the partial image based on a centroid of the bone of interest.

7. The image processing apparatus according to claim 1,
   wherein the processor is configured to function as a trained model that has undergone machine learning to output the likelihood of osteoporosis in response to an input of the bone part image or the partial image.

8. The image processing apparatus according to claim 1,
   wherein the processor is configured to function as a trained model that has undergone machine learning to output the likelihood of osteoporosis in response to an input of imaging conditions of the two radiation images in addition to the bone part image or the partial image.

9. The image processing apparatus according to claim 1,
   wherein the processor is configured to derive the likelihood of osteoporosis based on a statistical value of pixel values of the bone of interest included in the bone part image or the partial image.

10. The image processing apparatus according to claim 9,
    wherein the statistical value is at least one of an average value, a standard deviation, a maximum value, or a minimum value of pixel values in each of the bone of interest and a peripheral region of the bone of interest.

11. The image processing apparatus according to claim 10,
    wherein the processor is configured to derive the likelihood by performing a weighting operation on a plurality of statistical values among an average value, a standard deviation, a maximum value, and a minimum value of pixel values in each of the bone of interest and the peripheral region of the bone of interest.

12. The image processing apparatus according to claim 11,
    wherein the weight of a weighting operation is derived by machine learning.

13. The image processing apparatus according to claim 1,

wherein the processor is configured to issue a notification of the likelihood.

14. The image processing apparatus according to claim 1,
wherein the processor is configured to issue a warning in a case where the likelihood is equal to or greater than a predetermined threshold value.

15. The image processing apparatus according to claim 1,
wherein the processor is configured to:

    determine a priority of a treatment for osteoporosis based on the likelihood; and
    issue a notification of the priority.

16. The image processing apparatus according to claim 1,
wherein the processor is configured to remove scattered ray components included in the two radiation images to derive the bone part image.

17. The image processing apparatus according to claim 1,
wherein the processor is configured to:

    derive, in a soft region including only the soft part of the two radiation images, a characteristic of the soft part related to an attenuation of the radiation based on the two radiation images;
    derive, in a bone region including the bone part of the two radiation images, a characteristic of the soft part based on the characteristic of the soft part derived in the soft region around the bone region; and
    derive, as the bone part image, an image in which the bone part is emphasized based on the characteristic of the soft part in at least a region of the subject in the two radiation images.

18. The image processing apparatus according to claim 1,
wherein the bone of interest is a femur or a vertebra.

19. An image processing method comprising:

    via a computer,
    acquiring two radiation images acquired by imaging a subject including a bone part and a soft part with radiation having different energy distributions;
    deriving a bone part image including a microstructure inside the bone part based on the two radiation images; and
    deriving a likelihood of osteoporosis based on the bone part image or a partial image including a bone of interest in the bone part image.

20. An image processing program causing a computer to execute a process comprising:

    acquiring two radiation images acquired by imaging a subject including a bone part and a soft part with radiation having different energy distributions;
    deriving a bone part image including a microstructure inside the bone part based on the two radiation images; and
    deriving a likelihood of osteoporosis based on the bone part image or a partial image including a bone of interest in the bone part image.

# FIG. 1

IMAGE PROCESSING APPARATUS 10

IMAGE STORAGE SYSTEM 9

# FIG. 2

CPU 11

MEMORY 16

NETWORK I/F 17

18

STORAGE 13

IMAGE PROCESSING PROGRAM 12

DISPLAY 14

INPUT DEVICE 15

# FIG. 3

IMAGE PROCESSING APPARATUS — 10

IMAGE ACQUISITION UNIT — 21

SCATTERED RAY REMOVAL UNIT — 22

BONE PART IMAGE DERIVATION UNIT — 23

SEMENTATION UNIT — 24

LIKELIHOOD DERIVATION UNIT — 25

TRAINED MODEL — 25A

DISPLAY CONTROL UNIT — 26

# FIG. 4

# FIG. 5

EP 4 681 645 A1

## FIG. 6

60

61 65 66 65 66 67 63

| INPUT LAYER | → | CONVOLUTIONAL LAYER | → | POOLING LAYER | · · · · · | CONVOLUTIONAL LAYER | → | POOLING LAYER | → | FULLY-CONNECTED LAYER | → | OUTPUT LAYER |

62

## FIG. 7

40

OSTEOPOROSIS : PRESENT

41

42

43

## FIG. 8

25A

B(x,y) ──→ TRAINED MODEL ──→ P0

22

## FIG. 9

Gb    50

LIKELIHOOD OF
OSTEOPOROSIS: A %

51

## FIG. 10

```
        START
          │
          ▼                    ST1
   ACQUIRE RADIATION IMAGE
          │
          ▼                    ST2
    REMOVE SCATTERED RAY
        COMPONENT
          │
          ▼                    ST3
    DERIVE BONE PART IMAGE
   INCLUDING MICROSTRUCTURE
          │
          ▼                    ST4
       SEGMENTATION
          │
          ▼                    ST5
    DERIVE LIKELIHOOD OF
       OSTEOPOROSIS
          │
          ▼                    ST6
         DISPLAY
          │
          ▼
         END
```

FIG. 11

FIG. 12

## FIG. 13

PIXEL VALUE

POSITION

## FIG. 14

PIXEL VALUE

POSITION

## FIG. 15

## FIG. 16

| IMAGE PROCESSING APPARATUS | 10A |
|---|---|
| IMAGE ACQUISITION UNIT | 21 |
| SCATTERED RAY REMOVAL UNIT | 22 |
| BONE PART IMAGE DERIVATION UNIT | 23 |
| SEMENTATION UNIT | 24 |
| LIKELIHOOD DERIVATION UNIT | 25 |
| TRAINED MODEL | 25B |
| DISPLAY CONTROL UNIT | 26 |

## FIG. 17

OSTEOPOROSIS
: PRESENT

IMAGING
CONDITION

## FIG. 18

# FIG. 19

**IMAGE PROCESSING APPARATUS** — 10B

- IMAGE ACQUISITION UNIT — 21
- SCATTERED RAY REMOVAL UNIT — 22
- BONE PART IMAGE DERIVATION UNIT — 23
- SEMENTATION UNIT — 24
- LIKELIHOOD DERIVATION UNIT — 25
- DISPLAY CONTROL UNIT — 26

# FIG. 20

Gb

50A

LIKELIHOOD OF
OSTEOPOROSIS: A %

51

THERE IS HIGH
POSSIBILITY OF
OSTEOPOROSIS

52

# FIG. 21

Gb
50B

LIKELIHOOD OF
OSTEOPOROSIS: A %

51

TREATMENT
PRIORITY: HIGH

53

# FIG. 22

BONE PART IMAGE DERIVATION UNIT
23

REGION SPECIFICATION UNIT
31

CHARACTERISTIC DERIVATION UNIT
32

IMAGE DERIVATION UNIT
33

# FIG. 23

G1

G2

CL

CH

M1

K1

Cb0

Cb1

Cs1

Gb

Gs

# FIG. 24

```
                    ┌─────────┐
                    │  START  │
                    └─────────┘
                         │               ST11
         ┌───────────────▼───────────────┐
         │   DERIVE ATTENUATION IMAGE     │
         └───────────────┬───────────────┘
                         │               ST12
         ┌───────────────▼───────────────┐
         │  SPECIFY SOFT REGION AND BONE  │
         │            REGION              │
         └───────────────┬───────────────┘
                         │               ST13
         ┌───────────────▼───────────────┐
         │  DERIVE CHARACTERISTIC OF SOFT │
         │  PART COMPONENT IN SOFT REGION │
         └───────────────┬───────────────┘
                         │               ST14
         ┌───────────────▼───────────────┐
         │  DERIVE CHARACTERISTIC OF SOFT │
         │  PART COMPONENT IN BONE REGION │
         └───────────────┬───────────────┘
                         │               ST15
         ┌───────────────▼───────────────┐
         │    DERIVE INITIAL BONE PART    │
         │       ATTENUATION IMAGE        │
         └───────────────┬───────────────┘
                         │               ST16
         ┌───────────────▼───────────────┐
         │       DERIVE BONE PART         │
         │       ATTENUATION IMAGE        │
         └───────────────┬───────────────┘
                         │               ST17
         ┌───────────────▼───────────────┐
         │       DERIVE SOFT PART         │
         │       ATTENUATION IMAGE        │
         └───────────────┬───────────────┘
                         │               ST18
         ┌───────────────▼───────────────┐
         │     DERIVE BONE PART IMAGE     │
         └───────────────┬───────────────┘
                         │
                         ▼
                  TO STEP ST4
```

## INTERNATIONAL SEARCH REPORT

| International application No. |
|---|
| **PCT/JP2023/039909** |

### A. CLASSIFICATION OF SUBJECT MATTER

***A61B 6/00***(2024.01)i
FI:  A61B6/00 350Z; A61B6/00 360Z; A61B6/00 333

According to International Patent Classification (IPC) or to both national classification and IPC

### B. FIELDS SEARCHED

Minimum documentation searched (classification system followed by classification symbols)

A61B6/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | US 2008/0119719 A1 (THE REGENTS OF THE UNIVERSITY OF CALIFORNIA) 22 May 2008 (2008-05-22) paragraphs [0031]-[0101], etc., in particular, paragraphs [0032], [0039], [0049], [0065], [0073], etc. | 1-4, 9-14, 18-20 |
| Y | paragraphs [0031]-[0101], etc., in particular, paragraphs [0032], [0039], [0049], [0065], [0073], etc. | 5-8, 15-17 |
| X | JP 6-133223 A (FUJI PHOTO FILM CO., LTD.) 13 May 1994 (1994-05-13) paragraphs [0012], [0019], [0021]-[0042], etc. | 1-3, 9-14, 17-20 |
| Y | paragraphs [0012], [0019], [0021]-[0042], etc. | 4-8, 15-17 |
| Y | JP 2010-279418 A (IWATE MEDICAL UNIVERSITY) 16 December 2010 (2010-12-16) paragraphs [0002], [0015], [0025]-[0043], etc. | 4 |
| Y | JP 2009-207886 A (FUJIFILM CORPORATION) 17 September 2009 (2009-09-17) paragraphs [0001]-[0002], [0036]-[0058], etc. | 5-6 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **29 November 2023** | **12 December 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**EP 4 681 645 A1**

## INTERNATIONAL SEARCH REPORT

| International application No. |
| --- |
| **PCT/JP2023/039909** |

### C. DOCUMENTS CONSIDERED TO BE RELEVANT

| Category* | Citation of document, with indication, where appropriate, of the relevant passages | Relevant to claim No. |
| --- | --- | --- |
| Y | JP 9-248293 A (ASAHI CHEMICAL INDUSTRY CO., LTD.) 22 September 1997 (1997-09-22)<br>paragraphs [0001], [0031]-[0033], etc. | 7-8, 15 |
| Y | JP 2022-122131 A (FUJIFILM CORPORATION) 22 August 2022 (2022-08-22)<br>paragraphs [0039]-[0046], [0071]-[0079], etc. | 7-8, 16 |
| A | JP 2021-69791 A (FUJIFILM CORPORATION) 06 May 2021 (2021-05-06)<br>entire text, all drawings | 1-20 |
| A | JP 8-164129 A (ASAHI CHEMICAL INDUSTRY CO., LTD.) 25 June 1996 (1996-06-25)<br>entire text, all drawings | 1-20 |
| A | JP 2004-105738 A (GE MEDICAL SYSTEMS GLOBAL TECHNOLOGY COMPANY, LLC) 08 April 2004 (2004-04-08)<br>entire text, all drawings | 1-20 |

Form PCT/ISA/210 (second sheet) (January 2015)

## INTERNATIONAL SEARCH REPORT
### Information on patent family members

International application No.

**PCT/JP2023/039909**

| Patent document cited in search report | | | Publication date (day/month/year) | Patent family member(s) | | | Publication date (day/month/year) |
|---|---|---|---|---|---|---|---|
| US | 2008/0119719 | A1 | 22 May 2008 | WO | 2008/024790 | A2 | |
| JP | 6-133223 | A | 13 May 1994 | (Family: none) | | | |
| JP | 2010-279418 | A | 16 December 2010 | (Family: none) | | | |
| JP | 2009-207886 | A | 17 September 2009 | US paragraphs [0001]-[0004], [0058]-[0080], etc. | 2009/0202122 | A1 | |
| JP | 9-248293 | A | 22 September 1997 | (Family: none) | | | |
| JP | 2022-122131 | A | 22 August 2022 | US paragraphs [0059]-[0066], [0091]-[0099], etc. CN | 2022/0249013 114903504 | A1 A | |
| JP | 2021-69791 | A | 06 May 2021 | US entire text, all drawings | 2021/0128094 | A1 | |
| JP | 8-164129 | A | 25 June 1996 | (Family: none) | | | |
| JP | 2004-105738 | A | 08 April 2004 | US entire text, all drawings | 2004/0059223 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 113129286 A

- JP 2015043959 A